**Europäisches Patentamt**

(19) **European Patent Office**

**Office européen des brevets**

(11) Numéro de publication: **0 033 694**
**B1**

(12) **FASCICULE DE BREVET EUROPEEN**

(45) Date de publication du fascicule du brevet: **21.11.84**

(21) Numéro de dépôt: **81400140.0**

(22) Date de dépôt: **29.01.81**

(51) Int. Cl.³: **A 23 J 3/00,** A 23 C 9/152 // A61K37/18, A61K7/48, A23L1/30

(54) **Procédé pour l'obtention d'hydrolysats stabilisés de protéines animales, produits obtenus et application diététique et thérapeutique.**

(30) Priorité: **01.02.80 FR 8002279**

(43) Date de publication de la demande: **12.08.81 Bulletin 81/32**

(45) Mention de la délivrance du brevet: **21.11.84 Bulletin 84/47**

(84) Etats contractants désignés: **BE CH DE GB IT LI LU NL SE**

(56) Documents cités:
**FR-A- 489 303**
**FR-A-2 352 498**
**US-A-2 319 186**

**Le dossier contient des informations techniques présentées postérieurement au dépôt de la demande et ne figurant pas dans le présent fascicule.**

(73) Titulaire: **DISTRIVAL S.A.**
**B.P. 67 20, rue Cachin**
**F-50101 Cherbourg (FR)**

(72) Inventeur: **Noel, Bernard**
**24, rue Barbey d'Aurevilly**
**F-50700 Valognes (FR)**

(74) Mandataire: **Phélip, Bruno et al**
**c/o Cabinet Harlé & Phélip 21, rue de la Rochefoucauld**
**F-75009 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

## Description

La présente invention concerne un procédé pour l'obtention d'hydrolysats stabilisés de protéines animales. Elle concerne également les hydrolysats stabilisés de protéines animales ainsi obtenus ainsi que l'application de ces hydrolysats à titre d'aliments diététiques et de nutrition thérapeutique.

Les hydrolysats de protéines animales sont connus pour leur valeur nutritionnelle. Ces hydrolysats sont obtenus par digestion enzymatique de protéines animales. Après digestion enzymatique, les hydrolysats sont récupérés par des moyens classiques, tels que la filtration, la décantation et la centrifugation de manière à obtenir une fraction soluble exempte de toute contamination bactériologique. De tels hydrolysats sont ensuite concentrés à basse température, sous vide, en général jusqu'à une teneur en matière sèche de 60 à 70%.

Pour leur conservation, de tels hydrolysats sont aseptisés par acidification, puis conditionnés dans des emballages stériles à un pH compris entre 3 et 5, à l'abri de la lumière.

Pour plus de détails sur l'obtention de ces hydrolysats on pourra se référer par exemple aux ouvrages ci-après:
- TOMIYAMA, T. 1968, The Preparation of Fish Protein Concentrate by Autolysis. VIII Symposium sur les Matières Etrangères dans les Aliments. Varsovie. Un volume (709 p), pp. 385—398.
- HENRY, M. 1973, Alimentation, Flore intestinale et peau. VIIe Semaine Internationale de Dermopharmacologie pp 267—292 Centre Européen de Dermopharmacologie 181, Avenue Jean-Jaurès 69007 LYON.

Les hydrolysats concentrés de protéines animales obtenus selon le procédé ci-dessus, ont, par leur richesse en acides aminés (80 à 90% de l'azote total), une valeur nutritionnelle reconnue notamment par les travaux de:
- BALLESTER, E. Y. D. et MONCKEBERG, F. 1976, Enzymatic Fish Protein Hydrolysate Chemical Composition, Nutritive Value and Use as a Supplement to Cereal Protein, J. of Food Sc. 41, 1289—1292;
- CALET, C et BROUILLET, A. 1959, Intérêt de l'utilisation des autolysats dans l'alimentation du poulet. Les Industries de l'Alimentation Animale, n° 94, 29—32;
- DUBOS, R. 1962, Le rôle biologique de la flore digestive. Revue d'Immunologie, 26, 3, 97—111;
- ESCH, G. C. 1959, Recherches sur la valeur nutritive d'hydrolysats enzymatiques de protéines. Annals of Biochemistry and Experimental Medicine (India) XIX, 10, 241—244.

Cependant, ces hydrolysats concentrés constituent aussi des milieux de développement exceptionnels pour toute vie microbienne, dès qu'ils sont placés dans un milieu ambiant à des températures positives.

Il en résulte que leur utilisation en alimentation humaine nécessite des précautions de manipulation rigoureuses, et jusqu'à présent ils n'ont été utilisés que dans des applications cliniques.

D'autre part, au cours des opérations classiques de séchage pour l'obtention de ces hydrolysats sous forme de poudre, il se produit généralement une détérioration des éléments nutritionnels les plus intéressants, étant donnée la fragilité des acides aminés aux températures élevées, notamment aux températures supérieures à 70°C. En conséquence, les hydrolysats pulvérulents ainsi obtenus ne possèdent plus les propriétés actives de l'hydrolysat liquide, mentionnées dans les ouvrages cités précédemment.

A titre de documents illustrant l'état de la technique antérieure, on peut citer les suivants:

Le brevet FR—A-2 352 498, concerne un procédé et une installation pour la fabrication de concentrés de protéines de poissons par voie enzymatique utilisant les enzymes protéolytiques contenues dans les viscères de poissons. Ce brevet concerne en fait un exemple du produit de base mis en oeuvre dans le procédé selon l'invention, à savoir un hydrolysat de protéines de poissons.

Le brevet US—A-2 319 186 concerne du lait écrémé modifié et séché, mais n'enseigne pas l'application du lait à la protection de protéines animales.

Le brevet FR—A—489 303 concerne un procédé pour conserver l'albumine, qui consiste à mélanger l'albumine avec du lait. La description indique que le lait enveloppe les particules d'albumine et les protège contre toute transformation par la chaleur ou contre d'autres influences nuisibles. Le produit obtenu est séché à une température de 100°C ou plus, ou sous vide à une température moins élevée.

L'albumine est une des protéines du lait. Elle y est naturellement présente à raison d'environ 16%. Le fait d'ajouter de l'albumine soluble dans le lait ne modifie en rien la technique de séchage du lait qui contient déjà de l'albumine sensible à la chaleur, mais qui, par contre, est peu sensible à l'oxydation.

Au contraire, dans les hydrolysats mis en oeuvre selon l'invention, il n'y a plus d'albumine; ces hydrolysats sont constitués de micropeptides d'acides aminés libres, de vitamines et d'acides gras polyinsaturés, tous extrêmement sensibles à l'oxydation.

Alors que le brevet FR—A—489 303 propose l'incorporation dans le lait d'une substance qui y est soluble, la présente invention concerne un procédé de stabilisation, notamment à l'aide de lait, d'hydrolysats de protéines qui ne sont pas miscibles dans le lait. De tels hydrolysats, qui ont un caractère acide, provoqueraient la coagulation du lait, rendant ainsi impossible le séchage ultérieur.

Les problèmes posés par la conservation de l'albumine ne sont pas les mêmes que ceux des hydrolysats de protéines, qui sont extrêmement sensibles à l'oxydation.

Les problèmes ci-dessus se rencontrent d'une manière très particulière lorsqu'il s'agit d'hydrolysats à caractère acide.

On a maintenant trouvé un procédé pour stabiliser les hydrolysats de protéines animales, qui permet l'obtention de produits pouvant être manipulés sans risque de contamination ou d'oxydation et présentant les propriétés actives de l'hydrolysat de protéines de base.

Dans son principe, la présente invention concerne un procédé pour l'obtention d'hydrolysat stabilisé de protéines animales qui consiste:

a) à mélanger intimement sous forte agitation, une certaine quantité d'hydrolysat de protéines animales avec un produit liquide à base de lait en ajoutant progressivement l'hydrolysat au liquide agité, tout en maintenant la température entre 5 et 20°C,

b) à sécher le mélange résultant de manière que la température du mélange ne dépasse pas 65°C.

La quantité de produit à base de lait à mettre en oeuvre représentant au moins 50% de la composition totale de l'hydrolysat stabilisé.

Les hydrolysats de protéines animales, utilisés comme matière première dans le procédé selon la présente invention, sont obtenus par des techniques classiques, de préférence par autolyse ou par digestion enzymatique. Avantageusement, on utilise des hydrolysats concentrés ayant une teneur en matière sèche comprise entre 60 et 70%. On peut utiliser selon l'invention des hydrolysats de toute origine animale, tels que des hydrolysats de poisson, de pancréas, de sang, de lactoprotéines, etc.

Le produit à base de lait utilisé dans la présente invention, à titre d'agent de stabilisation, doit se présenter sous la forme liquide. Par "produit à base de lait" on désigne dans la présente description le lait entier ou écrémé, concentré ou non, les rétentats d'ultrafiltration du lait et autres produits analogues obtenus à partir du lait.

La qualité du produit à base de lait (entier, écrémé, concentré, rétentat d'ultrafiltration, etc.) n'est pas critique pour la mise en oeuvre de la première étape du procédé de l'invention. Le choix de la qualité est fonction de la destination du produit que l'on désire utiliser. Ainsi, par exemple, on utilisera de préférence un lait concentré écrémé pour fabriquer des produits diététiques de régime.

La première étape du procédé de l'invention consiste à mélanger intimement l'hydrolysat de protéines animales avec le produit à base de lait, de manière à réaliser un enrobage des molécules (ou microparticules) de l'hydrolysat.

Ce mélange est réalisé à une température comprise entre 5 et 20°C. En effet, on a trouvé que, seulement dans cette gamme de températures, l'hydrolysat et le produit à base de lait présentent en général les viscosités adéquates pour que se produise l'enrobage des microparticules d'hydrolysat par le fait. A une température inférieure à 5°C, le produit à base de lait est trop visqueux; par contre, à une température supérieure à 20°C, l'enrobage n'est pas satisfaisant.

La quantité de produit à base de lait à mettre en oeuvre dans le procédé de l'invention doit réaliser un enrobage satisfaisant des microparticules de l'hydrolysat. Cette quantité doit être d'au moins 50% de la composition totale de l'hydrolysat stabilisé.

Le mélange de l'hydrolysat et du produit à base de lait est réalisé par introduction progressive de l'hydrosat dans le produit à base de lait, maintenu sous agitation rapide, de manière à éviter toute coagulation des protéines du lait, et ensuite par homogénéisation du mélange résultant.

Selon une variante de mise en oeuvre du procédé de l'invention, on peut ajouter dans le produit à base de lait, avant l'étape de mélange, des sels de qualité alimentaire, tels que les phosphates monocalciques ou monosodiques. Ces sels facilitent les opérations ultérieures de séchage et empêchent en outre la coagulation des protéines du lait. De plus, cette variante permet d'adapter judicieusement, selon les besoins, la teneur en sels de l'hydrolysat stabilisé.

Selon une autre variante de l'invention, on peut ajouter, au mélange résultant de la première étape du procédé (contenant éventuellement des sels de qualité alimentaire), des éléments nutritifs, tels que des acides aminés particuliers, des vitamines, des acides gras, ou des agents stabilisants ou antioxydants primaires, tels que le butylhydroxytoluène (BHT), le butylhydroxyanisole (BHA), l'acide nitrique ou l'acide phosphorique, afin d'obtenir un produit final ayant des vertus alimentaires ou thérapeutiques particulières. Le choix des éléments à ajouter au mélange hydrolysat-produit à base de lait dépend de la qualité du produit final que l'on désire obtenir. De plus, ils doivent être tels qu'ils ne détruisent pas l'enrobage de l'hydrolysat par les protéines lactiques, réalisé au cours de la première étape du procédé de l'invention.

Le mélange produit à base de lait-hydrolysat, contenant éventuellement des sels de qualité alimentaire et/ou des éléments nutritifs, agents antioxydants ou agents stabilisants, est ensuite soumis à un séchage, par exemple par atomisation ou lyophilisation. Le séchage par atomisation doit être réalisé de telle sorte que les particules atomisées ne dépassent jamais la température de 65°C. En effet, à une température supérieure à 65°C, il se produit une dégradation des acides aminés de l'hydrolysat.

De préférence, le séchage est réalisé jusqu'à l'obtention d'une poudre ayant moins de 6% d'humidité.

Le séchage du mélange hydrolysat-produit à base de lait peut également être réalisé par lyophilisation dans des conditions appropriées pour obtenir un lyophilisat ayant une teneur en humidité avantageusement inférieure à 6%.

Les hydrolysats de protéines animales stabilisés selon le procédé de l'invention conservent intactes toutes les valeurs nutritionnelles de l'hydrolysat de protéines de départ. On a trouvé en effet que les hydrolysats obtenus selon le procédé de l'invention ont des teneurs en acides aminés au moins égales à 95% de la teneur en acides aminés de l'hydrolysat de départ. Ces hydrolysats stabilisés peuvent être utilisés tels quels ou en mélange avec des préparations aromatiques diverses, comme aliments diététiques ou comme aliments de nutrition thérapeutique.

Les hydrolysats stabilisés selon l'invention sont particulièrement appropriés pour les régimes hypocaloriques nécessaires pour soigner l'obésité, pour le traitement de certaines hyperlipoprotéinémies, d'états comportant habituellement une carence protidique.

Dans le cas de traitement d'états comportant habituellement une carence protidique, on a traité 20 malades de catégories différentes:

2 malades du 3ème âge présentant une malnutrition, dénutrition ou anorexie;

1 malade du cancer;

3 malades présentant des désordres nutritionnels de l'éthylisme;

5 malades présentant des ulcères variqueux ou escarres.

Le groupe témoin était constitué de:

10 personnes souffrant de malnutrition;

2 malades du cancer;

3 malades présentant des désordres nutritionnels de l'éthylisme;

5 malades présentant des ulcères variqueux ou escarres.

Le produit de l'invention emballé en sachets étanches de 26 g a été administré à raison de deux sachets par jour pendant 20 jours de suite. L'étude clinique a montré une amélioration de l'état général et de l'état psychique très largement supérieure chez les patients traités avec le produit de l'invention par rapport aux patients témoins. Biologiquement, la protidémie était plus élevée dans le groupe de patients traités avec le produit de l'invention (5,4%) que chez les patients témoins (2,7%). L'introduction du produit de l'invention dans la diététique des hyperlipoprotéinémies a donné des résultats statistiquement valables pour la correction de certains troubles lipidiques.

Dans le groupe des hyperlipoprotéinémies 2A on a obtenu sur 6 patients une amélioration du bilan lipidique sur le plan quantitatif et qualitatif. Dans les hyperlipoprotéinémies mixtes, sur 6 cas on a également obtenu une amélioration quantitative et qualitative à la fois sur le cholestérol total et de l'HDL cholestérol mais aussi sur les triglycérides. Dans les hyperlipoprotéinemies de type IV le résultat était particulièrement intéressant avec une baisse nette du chiffre des triglycérides et une bonne stabilité des résultats chez des sujets dont on connaissait habituellement l'instabilité des taux de triglycérides.

Par ailleurs le produit selon l'invention a été donné à des patients obèses. On a trouvé que l'hydrolysat stabilisé selon l'invention était un excellent complément dans les régimes hypocaloriques tant par ses effets bénéfiques sur l'amaigrissement, l'état général, le dynamisme du patient, que par sa très bonne acceptabilité et ses effets sur la satiété.

En général, les hydrolysats stabilisés selon l'invention sont conditionnés en sacs étanches. Ils peuvent également être conditionnés sous la forme de gélules, de comprimés, de dragées, de sondes injectables ou toutes autres formes qui permettent leur utilisation en nutrition thérapeutique. Les dragées sont avantageusement obtenues après compression de la poudre d'hydrolysat stabilisé par enrobage avec une substance d'enrobage classique, par exemple un sucre, tel que le glucose. Elles constituent une forme de conditionnement très appropriée pour les hydrolysats stabilisés selon l'invention.

L'invention concerne aussi des compositions cosmétiques contenant une quantité efficace d'un hydrolysat de protéines, stabilisé conformément à l'invention, en association avec un excipient convenant à ce type d'application.

Les hydrolysats stabilisés selon l'invention peuvent par exemple être solubilisés dans un polyalkylène glycol, en particulier dans le polyéthylène glycol, pour servir de base à la préparation de sirops. De tels produits peuvent trouver application en cosmétique.

La présente invention va être maintenant illustrée par les exemples ci-après.

Exemple 1

On a utilisé comme hydrolysat de départ un hydrolysat enzymatique de poisson. Cet hydrolysat de poisson a été obtenu après digestion enzymatique de poisson de mer frais à une température comprise entre 50 et 60°C et à un pH compris entre 3 et 5,5, pendant 6 heures. Cet hydrolysat a ensuite été partiellement déshuilé par des procédés physiques (décantation, centrifugation, filtration) puis concentré sous vide à une température de 65°C de façon à obtenir un produit ayant la composition suivante:

Extrait sec: 60%
pH: 4
Analyse chimique (% en poids)
Azote total: 13, dont acides aminés libres 5,3
Matière grasse: 8
Cendres: 10,75

Comme produit à base de lait, on a utilisé du lait écrémé et concentré sous vide jusqu'à 50% d'extrait sec, la concentration ayant été réalisée à 55°C. Ce lait ainsi concentré a ensuite été refroidi dans un échangeur tubulaire, jusqu'à une température de 15°C, et envoyé ensuite dans une cuve graduée munie d'un agitateur rapide de type "Turbo-mix". On a ajouté au lait concentré, à raison de 1 litre pour 100 litres, une solution à 10% de phosphate monocalcique. On a ensuite procédé à l'incorporation progressive de l'hydrosat dans le lait concentré, sous agitation rapide, et on a obtenu un mélange final comprenant, en poids par rapport à une base sèche, 32% d'hydrolysat de poisson. Le mélange ainsi obtenu a été envoyé sur un homogénéisateur basse pression, à filière, refroidi de façon à ce que le mélange ne dépasse pas 20°C. Le produit homogénéisé a ensuite été envoyé sur une tour de séchage verticale et atomisé dans la chambre alimentée en air sec à 155°C. Le séchage a été réalisé de façon que les particules atomisées soient recueillies en bas de chambre à une température inférieure à 60°C. L'hydrolysat pulvérulent ainsi obtenu a ensuite été ensaché aseptiquement dans des emballages étanches à une humidité de 5,5%.

Exemple 2

On a utilisé dans cet exemple un hydrolysat de pancréas. Cet hydrolysat a été obtenu après digestion enzymatique de pancréas bovin selon le mode opératoire décrit à l'exemple 1. Après concentration, l'hydrolysat, contenant 35% d'extrait sec environ, avait un pH de 4,5.

Le produit à base de lait utilisé était du lait écrémé concentré sous vide à 57% d'extrait sec à une température de 58°C. Ce lait a ensuite été refroidi dans un échangeur tubulaire jusqu'à une température de 12°C et envoyé sur un mélangeur-doseur en continu. On a introduit dans ce mélangeur-doseur l'hydrolysat de pancréas ci-dessus en une quantité telle que l'hydrolysat stabilisé résultant contenait 30% d'hydrolysat.

Le mélange ainsi obtenu a ensuite été lyophilisé dans un dispositif classique de lyophilisation. Après passage dans le lyophilisateur, le produit desséché obtenu a été réduit en poudre dans un émietteur à produits fragiles, puis conditionné en sacs étanches jusqu'à une humidité de 5%.

Revendications

1. Procédé pour l'obtention d'un hydrolysat stabilisé de protéines animales, caractérisé en ce qu'il consiste:

a) à mélanger intimement sous forte agitation, une certaine quantité d'hydrolysat de protéines animales avec un produit liquide à base de lait en ajoutant progressivement l'hydrolysat au liquide agité, tout en maintenant la température entre 5 et 20°C,

b) à sécher le mélange résultant de manière que la température du mélange ne dépasse pas 65°C, la quantité de produit à base de lait à mettre en oeuvre représentant au moins 50% de la composition totale de l'hydrolysat stabilisé.

2. Procédé selon la revendication 1, caractérisé en ce que le produit à base de lait est du lait concentré, entier ou écrémé, un rétentat d'ultrafiltration ou autre produit analogue obtenu à partir du lait.

3. Procédé selon l'une des revendications 1 ou 2, caractérisé en ce que l'hydrolysat mis en oeuvre est un hydrolysat enzymatique de poissons, de pancréas, de sang, de lactoprotéines ou autre hydrolysat de protéines d'origine animale.

4. Procédé selon l'une quelconque des revendications 1 à 3, caractérisé en ce que les hydrolysats enzymatiques ont une teneur en matière sèche comprise entre 60 et 70%.

5. Procédé selon l'une quelconque des revendications 1 à 4, caractérisé en ce que, avant l'étape de mélange, on ajoute au produit à base de lait des sels de qualité alimentaire, tels que des phosphates monocalciques et/ou monosodiques.

6. Procédé selon l'une quelconque des revendications 1 à 5, caractérisé en ce qu'on ajoute au mélange résultant de l'étape (a) des éléments nutritifs, tels que des acides aminés, des vitamines, des acides gras ou des agents stabilisants ou antioxydants primaires.

7. Procédé selon l'une quelconque des revendications 1 à 6, caractérisé en ce que l'hydrolysat est mis en oeuvre à un pH compris entre 3 et 5.5.

8. Procédé selon l'une quelconque des revendications 1 à 7, caractérisé en ce que le séchage est réalisé par atomisation ou par lyophilisation.

9. Hydrolysat stabilisé de protéines animales, obtenu par le procédé selon l'une quelconque des revendications 1 à 8.

10. Hydrolysat de protéines animales selon la revendication 9, caractérisé en ce qu'il se présente sous la forme d'une poudre ayant une teneur en humidité inférieure à 6% en poids.

11. Aliments diététiques ou de nutritions thérapeutique, caractérisés en ce qu'ils contiennent une quantité efficace d'hydrolysat stabilisé selon l'une des revendications 9 ou 10.

12. Aliments selon la revendication 11, présentés sous forme de dragées obtenues après compression de la poudre d'hydrolysat stabilisé et enrobage avec une substance d'enrobage classique, telle que le glucose.

13. Aliments selon l'une des revendications 11 ou 12 dont la teneur en acides aminés est au moins égale à 95% de la teneur en acides aminés de l'hydrolysat de départ.

14. Composition cosmétique contenant une quantité efficace d'hydrolysat stabilisé selon l'une des revendications 9 ou 10, en association avec un excipient usuel.

15. Composition cosmétique selon la revendication 14 présentée sous forme de sirop conditionné dans un polyalkylène glycol, tel que le polyéthylène glycol.

Patentansprüche

1. Verfahren zur Herstellung eines stabilisierten Hydrolysats von tierischen Proteinen, dadurch gekennzeichnet, daß es besteht aus:

(a) gründlichem Mischen unter starkem Rühren einer bestimmten Menge eines Hydrolysats von tierischen Proteinen mit einem flüssigen Produkt auf Basis von Milch, indem man das Hydrolysat nach und nach zu der gerührten Flüssigkeit gibt und bei allem die Temperatur zwischen 5 und 20°C hält,

(b) Trocknen des erhaltenen Gemisches derart, daß die Temperatur des Gemisches nicht 65°C überschreitet, wobei die einzusetzende Menge des Produkts auf Basis von Milch mindestens 50% der Gesamtzusammensetzung des stabilisierten Hydrolysats ausmacht.

2. Verfahren nach Anspruch 1, dadurch gekennzeichnet, daß das Produkt auf Basis von Milch konzentrierte, Voll- oder entrahmte Milch, ein Ultrafiltrations-Retentat oder ein anderes analoges Produkt ist, das ausgehend von Milch erhalten wurde.

3. Verfahren nach einem der Ansprüche 1 oder 2, dadurch gekennzeichnet, daß das eingesetzte Hydrolysat ein enzymatisches Hydrolysat von Fischen, von Pankreas, von Blut, von Lactoproteinen oder ein anderes Hydrolysat von Proteinen tierischen Ursprungs ist.

4. Verfahren nach irgendeinem der Ansprüche 1 bis 3, dadurch gekennzeichnet, daß die enzymatischen Hydrolysate einen Trockensubstanzgehalt zwischen 60 und 70% haben.

5. Verfahren nach irgendeinem der Ansprüche 1 bis 4, dadurch gekennzeichnet, daß man vor der Mischstufe dem Produkt auf Basis von Milch Salze von Nahrungsmittelqualität zusetzt, wie Monocalcium- und/oder Mononatriumphosphate.

6. Verfahren nach irgendeinem der Ansprüche 1 bis 5, dadurch gekennzeichnet, daß man dem in Stufe (a) erhaltenen Gemisch Nährelemente zusetzt, wie Aminosäuren, Vitamine, Fettsäuren, oder stabilisierende Mittel oder primäre Antioxidantien.

7. Verfahren nach irgendeinem der Ansprüche 1 bis 6, dadurch gekennzeichnet, daß das Hydrolysat bei einem pH zwischen 3 und 5,5 eingesetzt wird.

8. Verfahren nach irgendeinem der Ansprüche 1 bis 7, dadurch gekennzeichnet, daß die Trocknung durch Versprühen oder durch Lyophilisieren durchgeführt wird.

9. Stabilisiertes Hydrolysat von tierischen Proteinen, erhalten nach dem Verfahren nach irgendeinem der Ansprüche 1 bis 8.

10. Hydrolysat von tierischen Proteinen nach Anspruch 9, dadurch gekennzeichnet, daß es in Form eines Pulvers mit einem Feuchtigkeitsgehalt von weniger als 6 Gewichtsprozent vorliegt.

11. Diätetische Nahrungsmittel oder therapeutische Nährmittel, dadurch gekennzeichnet, daß sie eine wirksame Menge eines stabilisierten Hydrolysats nach einem der Ansprüche 9 oder 10 enthalten.

12. Nahrungsmittel nach Anspruch 11 in Form von Dragées, erhalten durch Pressen eines Pulvers des stabilisierten Hydrolysats und Umhüllen mit einer herkömmlichen Umhüllungssubstanz, wie Glucose.

13. Nahrungsmittel nach einem der Ansprüche 11 oder 12, bei denen der Gehalt an Aminosäuren mindestens gleich 95% des Gehalts an Aminosäuren des Hydrolysats ist, von dem ausgegangen wird.

14. Kosmetische Zusammensetzung, enthaltend eine wirksame Menge eines stabilisierten nach einem der Ansprüche 9 oder 10 in Kombination mit einem üblichen Excipienten.

15. Kosmetische Zusammensetzung nach Anspruch 14 in Form eines in einem Polyalkylenglykol, wie Polyethylenglykol, konditionierten Sirups.

**Claims**

1. Process for obtaining a stabilized hydrolysate of animal proteins, characterized in that it comprises:
   a) intimately mixing, with vigorous stirring, a certain amount of hydrolysate of animal proteins with a liquid product based on milk by gradually adding the hydrolysate to the stirred liquid while maintaining the temperature in the range of 5 to 20°C and
   b) drying the resulting mixture in such a way that the temperature of the mixture does not exceed 65°C, the amount of product based on milk which is to be employed representing at least 50% of the total composition of the stabilized hydrolysate.

2. Process according to claim 1, characterized in that the product based on milk is evaporated, whole or skimmed milk, a product retained from ultrafiltration or some other similar product obtained from milk.

3. Process according to one of claims 1 or 2, characterized in that the hydrolysate employed is an enzymatic hydrolysate of fish, pancreas, blood or lactoproteins, or some other hydrolysate of proteins of animal origin.

4. Process according to any one of claims 1 to 3, characterized in that the enzymatic hydrolysates have a solid content of between 60 and 70%.

5. Process according to any one of claims 1 to 4, characterized in that, before the mixing step, salts of foodstuff grade, such as monocalcium and/or monosodium phosphate salts, are added to the product based on milk.

6. Process according to any one of claims 1 to 5, characterized in that to the mixture resulting from step (a) there are added nutrient compounds such as aminoacids, vitamins of fatty acids, or primary stabilizers or antioxidants.

7. Process according to any one of claims 1 to 6, characterized in that the hydrolysate is employed at a pH from 3 to 5.5.

8. Process according to any one of claims 1 to 7, characterized in that the drying is effected by spray drying or freeze-drying.

9. Stabilized hydrolysate of animal proteins, obtained by the process according to any one of claims 1 to 8.

10. Hydrolysate of animal proteins according to claim 9, characterized in that it is a powder having a moisture content of less than 6% by weight.

11. Dietetic foodstuffs or foodstuffs for therapeutic nutrition, characterized in that they contain an effective amount of a stabilized hydrolysate according to one of claims 9 or 10.

12. Foodstuffs according to claim 11, in unit dosage form which are coated pills obtained by compressing the stabilized hydrolysate powder and coating with a conventional coating substance such as glucose.

13. Foodstuffs according to one of claims 11 or 12, wherein the aminoacid content is at least 95% of the aminoacid content of the hydrolysate starting material.

14. Cosmetic composition containing an effective amount of a stabilized hydrolysate according to one of claims 9 or 10 in association with a conventional excipient.

15. Cosmetic composition according to claim 14 presented in the form of a syrup comprising a polyalkylene glycol, such as polyethylene glycol.